# EUROPEAN PATENT SPECIFICATION

(11) **EP 4 146 475 B1**
(45) Date of publication and mention of the grant of the patent: **25.09.2024**
(21) Application number: 21728983.4
(22) Date of filing: 05.05.2021
(51) Int. Cl.: B29C 64/118, B29C 64/124, B33Y 10/00, B33Y 70/00, B29C 39/24, B29C 33/52, B29C 39/42, A61F 2/04, A61F 2/82, A61F 9/007

(54) **HIGH RESOLUTION 3D PRINTING PROCESS OF COMPLEX STRUCTURES**
HOCHAUFLÖSENDER 3D-DRUCKPROZESS VON KOMPLEXEN GEBILDEN
PROCESSUS D'IMPRESSION 3D À HAUTE RÉSOLUTION DE STRUCTURES COMPLEXES

(30) Priority: 07.05.2020 CH 5462020; 07.05.2020 IT 202000010219
(43) Date of publication of application: 15.03.2023
(73) Proprietor: 4Devices Medical Sagl, 6596 Gordola (CH)
(72) Inventor: DE MARCO, Carmela, 5000 Aarau (CH)
(74) Representative: Fabiano, Piero
(86) International application number: PCT/IB2021/053788
(87) International publication number: WO 2021/224816

(56) References cited:
- EP-A1- 3 348 384
- EP-A1- 3 348 384
- CN-A- 108 478 879
- CN-A- 108 478 879
- US-A1- 2020 062 877
- US-A1- 2020 062 877

## Description

### Field of the invention

The present invention concerns a three-dimensional printing process of high resolution, preferably medical, devices with complex geometries.

### Known art

As known, many medical applications in which, for example, an artificial or natural stoma must be held open permanently (non-resorbable) or temporarily (resorbable) in a membrane, wall, etc. require the insertion of a small medical device, such as a stent. An application example is the treatment of hydrocephalus by inserting a loop/stent.

A further use example of very small devices is represented by stents for the treatment of glaucoma, or scaffolds for tissue engineering or even stents used for the anastomoses of small vessels.

Such medical devices should be able to reach very small dimensions, could, for example, be inscribed in cylindrical tubular elements of less than 1 cm in length or of less than 5 mm in diameter.

The Applicant has observed that in the biomedical field, a vast class of biocompatible polymeric materials (polylactic acid (PLA), polyglycolic acid (PGA) and their copolymers such as polylactic-co-glycolic acid (PLGA), polycaprolactone (PCL), polyvinyl alcohol (PVA), thermoplastic polyurethanes (TPU), polyester urethanes (PEU) and polypropylene carbonates (PPC)) are of interest for the implementation of medical devices, such as for example stents, although the current three-dimensional molding techniques are limited to making cylindrical shapes with diameters of no more than 4-5 mm.

The Applicant has observed that for such type of devices, for example in the medical field, not all the materials can be printed with the current three-dimensional or 3D printing techniques. In particular, the thermoplastic polymers are generally printed with the Fused Deposition Modelling (FDM) technique, which however has the drawback of having low printing resolutions.

The current 3D printing techniques are direct techniques, i.e. are based on the stratification of the material (additive manufacturing) and this means that complex structures cannot be created.

It is possible to print some thermosetting materials through three-dimensional printing techniques, such as stereolithography or Continuous Digital Light manufacturing (cDLM).

US2020/0062877 discloses curable formulations suitable for manufacturing ceramic, polymeric, metal or composite products such as medical devices. A method is disclosed wherein a patterned structure (positive mold) is formed from a curable formulation by means of a three-dimensional printing method. This positive mold is sacrificial, i.e., it is coated with alumina ceramic slurry and then the coated positive mold is immersed in water to leach away the printed article prototype. The so obtained negative mold is filled with melted metal solder and after cooling the metal is solidified and the final product is obtained which reproduces the geometries of the 3D printed polymeric patterned structure. However, the Applicant has observed that very few thermosetting materials can be printed with such techniques due to the very high density of energy generally required by the thermosetting resins for their curing.

The Applicant has thus addressed the problem of implementing a three-dimensional printing process of high resolution medical devices with complex geometries.

The Applicant has further addressed the problem of implementing a three-dimensional printing process of high resolution, preferably medical, devices with complex geometries which can use different materials with respect to the thermoplastic or thermosetting materials used up to now by the known art.

### Summary of the invention

Thus, in a first aspect, the invention concerns a three-dimensional printing process of high resolution, preferably medical, devices with complex geometries, comprising the steps of:
- printing a model with a three-dimensional printing method by using a three-dimensional printer; said model positive reproducing the medical device to be made;
- said model being printed of a first water-soluble polymer or aqueous solutions;
- covering said model with a layer of material insoluble to a solution able to dissolve said first soluble polymer; said covering step making a shell of solid mold provided with a surface comprising empty interstitial spots;
- infiltrating an amount of water or aqueous solution into said solid mold through said empty interstitial spots so that to dissolve said model and to make a mold cavity negative reproducing said model;
- infiltrating into the mold cavity, through the outer walls of said mold, a second liquid polymer; said material having a melting temperature greater than the melting temperature of said second liquid polymer and/or a difference in the Hildebrand solubility parameter greater than or equal to 2 cal^{1/2} cm^{-3/2} with respect to that of said second liquid polymer; said step of infiltrating into the mold cavity occurs by depositing the liquid polymer on the outer walls of said mold, wherein the second liquid polymer solidifies or hardens in the mold cavity and the device is formed";
- removing the mold by releasing the product obtained and created in said second polymer.

In the context of the present invention, "second liquid polymer" can be understood as either a thermoplastic polymer in solution, as a thermoplastic polymer above its melting temperature Tm, or as a thermosetting prepolymer.

In the aforesaid aspect, the present invention can have at least one of the preferred characteristics described hereunder.

Preferably, the model is printed with a water-soluble polymer selected between thermoplastics or thermosets.

Advantageously, the first soluble polymer comprises at least one among polyvinyl alcohol, dimethylacrylamide (DMA), methacrylic acid (MA) and its esters, methacrylic anhydride (MAA), polyvinylpyrrolidone (PVP), succinic acid (SAA) and its esters or a combination thereof.

Preferably, the model is completely covered with a layer of material of a maximum thickness of 2 cm.

Preferably, the covering material has a melting temperature greater than the melting temperature of said second polymer or said prepolymer mixture.

Conveniently, said covering material is selected among wax, silicone-based elastomers or perfluoropolyethers.

Advantageously, the second polymer or prepolymer mixture is a biocompatible shape-memory polymer.

Preferably, the second biocompatible shape-memory polymer is selected between a one-way biocompatible shape-memory polymer or a two-way biocompatible shape-memory polymer.

Preferably, the second biocompatible shape-memory polymer is Norland Optical Adhesive 63^{™}.

Conveniently, in the step of infiltrating into the mold cavity a second liquid polymer through the outer walls of the mold, the infiltration occurs by capillarity.

Preferably, in the step of infiltrating into the mold cavity a second liquid polymer through the outer walls of the mold, the mold and the second liquid polymer deposited thereon are subjected to the action of a vacuum source which creates a vacuum inside the mold cavity.

Conveniently, in the step of infiltrating into the mold cavity a second liquid polymer through the outer walls of the mold, the infiltration occurs by injecting the second liquid polymer into the mold cavity.

Preferably, in the step of removing the mold, the mold is immersed into an organic solvent so that to be dissolved.

Alternatively, in the step of removing the mold, the latter is removed mechanically.

Alternatively, in the step of removing the mold, said mold is subjected to the heat generated by a heat source to melt it.

Further characteristics and advantages of the invention will become clearer in the detailed description of some preferred, but not exclusive, embodiments of a process according to the present invention.

### Brief description of the drawings

Such description will be exposed hereunder with reference to the accompanying drawings provided by way of example only and thus not limiting, in which:
- figure 1 shows a block diagram of the printing process of high resolution, preferably medical, devices with complex geometries, according to the present invention; and
- figure 2 is a schematic view of some steps of a printing process of high resolution, preferably medical, devices with complex geometries according to the present invention.

### Detailed description of embodiments of the invention

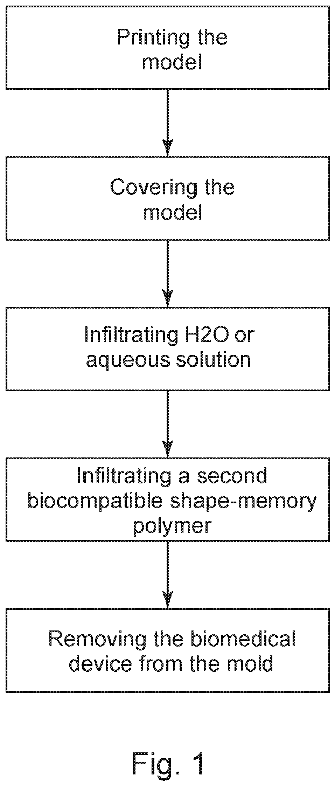

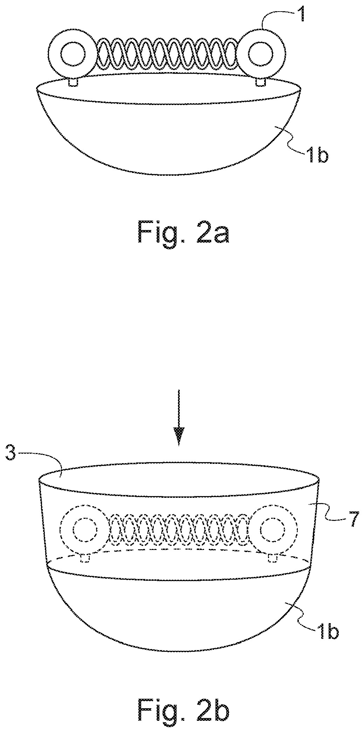

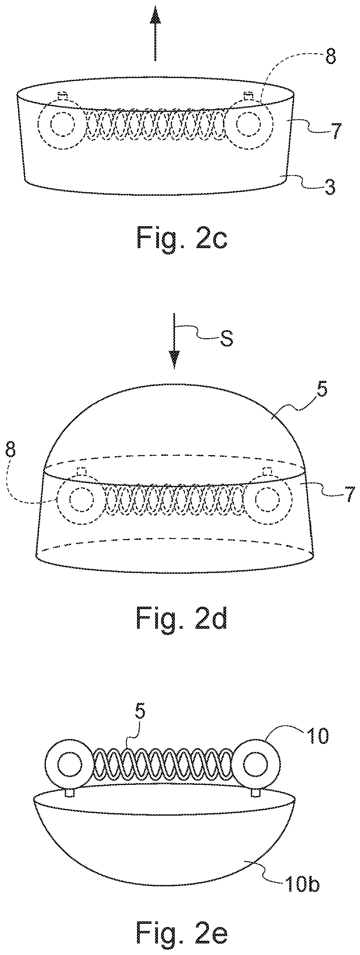

With reference to the figures and in particular to figures 1 and 2, the steps of a printing process of high resolution, preferably medical, devices 10 with complex geometries according to the present invention, are shown.

The process starts with the implementation of a model 1 which positive reproduces the high resolution, preferably medical, device 10 with complex geometries to be made, fig. 2a.

The model 1 is made by means of three-dimensional printing techniques. Preferably, the model 1 is made by means of three-dimensional printing techniques which use nano- or microscale or milliscale printers.

For example, the model 1 is made with printers that use the Fused Deposition Modelling (FDM) technique or Continuous Digital Light manufacturing (cDLM) and other techniques of stereolithography and molding.

Preferably, the model 1 is made of a first water-soluble polymer 2 or aqueous solutions with a pH of less than 6 or greater than 8. Preferably, in aqueous solutions having a pH of between 3 and 6 or of between 8 and 12. Aqueous solutions adapted for the purpose are basic solutions with a maximum of 1M NaOH or acidic solutions with a maximum of 1M HCl.

The model 1 is printed with a water-soluble polymer 2 or aqueous solutions selected between thermoplastics or thermosets.

Preferably, the first soluble polymer 2 comprises at least one among polyvinyl alcohol, dimethylacrylamide (DMA), methacrylic acid (MA) and its esters, methacrylic anhydride (MAA), polyvinylpyrrolidone (PVP), succinic acid (SAA) and its esters or a combination thereof.

The model 1 is printed together with a support 1b which, in the embodiment shown in figures 2a-2b, appears hemispherical in shape with the flat base facing upwards, i.e. facing the model 1 so that to provide a surface for containing the covering material 3.

Whenever the model 1 is printed with a thermoplastic polymer, once the model 1 has been printed it is left to cool, and generally a few minutes are sufficient.

Alternatively, whenever a thermosetting polymer has been printed with stereolithography or with Continuous Digital Light Manufacturing (cDLM) techniques, the model 1 is subjected to a source of UV rays for at least 5 minutes or to a source of heat.

Subsequently, it is thus possible to proceed to a step of covering the model 1 with a layer of material 3 insoluble to a solution able to dissolve the first soluble polymer 2.

The covering step, shown in figure 2b, concerns the whole outer surface of the model 1 and allows to make a shell of solid mold 7 provided with an outer surface comprising empty interstitial spots.

In the embodiment shown in figures 1, 2a-2e, the covering material 3 forms a solid mold 7 consisting of a cylindrical body around the model 1, delimited by the support 1b at the bottom.

In order to make the covering step, a layer of material 3 of at least 1 mm thickness, preferably of at least 2 mm, anyhow a layer of substance 3 equal to maximum 2 cm thickness, is deposited over the entire outer surface of the model.

In order for the solid mold 7 to withstand the subsequent infiltration step, the substance 3, which substantially forms the outer walls of the mold, must have a melting temperature greater than the melting temperature of the second liquid, preferably biocompatible, polymer 5 which will subsequently be infiltrated so that to make the final product, i.e. the medical device 10.

Alternatively, always for such purpose, the substance 3, which substantially forms the outer walls of the mold, must have a difference in the Hildebrand solubility parameter greater than or equal to 2 cal^{1/2}cm^{-3/2} with respect to that of the second liquid polymer 5.

In the embodiment shown, in particular in figures 2b-2d, the covering material 3 has a melting temperature greater than the melting temperature of the second liquid polymer 5 which will subsequently be infiltrated so that to make the final product.

Preferably, the material 3 is selected among wax, silicone-based elastomers or perfluoropoly ethers.

Preferably, in the embodiment shown in figures 2b-2d, the material 3 is wax.

Once the mold 7 has been made, as shown in figures 2b-2d, it is possible to immerse the solid mold in water or in a weakly acidic or basic aqueous solution so that the water or solution crosses the empty interstitial spots of the surface of the mold 7 and penetrates therein, thus dissolving the model 1.

By way of example, basic solution is, for example, understood as a solution of 1M NaOH.

Acidic solution is, for example, understood as a solution of 1M HCl maximum.

By dissolving the model 1, a mold cavity 8 negative reproducing the model 1 is thus made and consequently, always negative, the medical device 10 to be made, figure 2c.

The support 1b made of the same material and integral with the model 1 is also dissolved together with the model 1.

Thus, a second liquid polymer 5 is infiltrated into the mold cavity 8 through the outer walls of the mold 7.

In the present description, "second liquid polymer" can be understood as either a thermoplastic polymer in solution, as a thermoplastic polymer above its melting temperature Tm, or as a thermosetting polymer.

Preferably, the second polymer 5 is a biocompatible polymer.

In the embodiment shown in figures 2d-2e, the second polymer 5 is a biocompatible shape-memory polymer and is at the liquid state when being infiltrated.

Preferably, the second biocompatible shape-memory polymer 5 is selected between a one-way biocompatible shape-memory polymer, generally known as "one-way" in literature, or a two-way biocompatible shape-memory polymer, generally known as "two-way" in literature.

In the embodiment shown in figure 2, the second biocompatible shape-memory polymer comprises Norland Optical Adhesive 63^{™}.

In the embodiment shown in figure 2, the second biocompatible shape-memory polymer is Norland Optical Adhesive 63^{™}.

To infiltrate into the mold cavity 8 the second liquid polymer 5, the latter is deposited onto the outer walls of the mold 7.

Whenever the angle of contact between the liquid polymer 5 and the empty interstitial spot is less than 90°, the polymer enters the mold cavity 8 through the interstitial spot by simple capillarity.

Alternatively, whenever the angle of contact between the liquid polymer 5 and the empty interstitial spot is greater than or equal to 90°, an injection system is used for the infiltration or the mold 7 and the second liquid polymer 5 deposited thereon are subjected to the action of a vacuum generator which creates a depression inside the mold cavity 8 to attract the second liquid polymer 5 therein.

Whenever the second biocompatible polymer 5 is a thermoplastic polymer, it is possible to remove the mold thus releasing the product thus obtained and created in the second biocompatible polymer 5 once the second biocompatible polymer 5 is completely infiltrated into the mold cavity and left to polymerize, typically at room temperature and for a period of no less than 20 minutes.

Alternatively, whenever the second biocompatible polymer 5 is a thermosetting polymer, it is necessary to subject it to UV irradiation.

In the embodiment shown in figure 2, the preferably medical device 10 is thus made in Norland Optical Adhesive 63.

The step of removing the mold, figure 2e, can occur by breaking the mold 7 and by extracting the finished device 10, preferably medical, for example typically when the mold 7 is an elastomer.

In fact, in this case, the mold 7 can be delicately cut and peeled off since the softness of the material it is made of allows its removal without compromising the final object (finished device 10) and its components.

Alternatively, the step of removing the mold 7 can occur by dissolving the latter, i.e. by immersing the mold 7 infiltrated by the second liquid polymer 5 into an organic solvent which will dissolve the mold 7 without affecting the polymer 5.

Preferably, the organic solvent has a difference in the Hildebrand solubility parameter of an absolute value of less than 2 cal^{1/2} cm^{-3/2} with respect to that of said second liquid polymer 5 and a difference in the Hildebrand solubility parameter of an absolute value greater than 2 cal^{1/2} cm^{-3/2} with respect to that of said mold 7.

Or, still alternatively, by subjecting the mold 7 to heat, for example by placing the mold 7 in an appropriate furnace.

The temperature to which the furnace should be set and the time of residence therein depend on the material 3 with which the mold 7 is made.

Whenever the polymer 5 is a thermoplastic, the temperature of the furnace must be lower than the melting temperature of the polymer 5, whenever the polymer 5 is a thermoset, the temperature of the furnace must instead be lower than the softening temperature of the thermoset.

The medical device obtained is shown in figure 2e constrained to a support 10b made of the same shape-memory polymer 5 as that of the device 10.

The support 10b is subsequently removed.

Several changes can be made to the embodiments described in detail, all anyhow remaining within the protection scope of the invention, defined by the following claims.

## Claims

1. Process of printing high resolution, preferably medical, devices (10) with complex geometries, comprising the steps of:
- printing a model (1) with a three-dimensional printing method by using a three-dimensional printer; said model (1) positive reproducing the medical device (10) to be made;
- said model (1) being printed of a first water-soluble polymer (2) or aqueous solutions;
- covering said model (1) with a layer of material (3) insoluble to a solution able to dissolve said first soluble polymer (2); said covering step making a shell of solid mold (7) provided with a surface comprising empty interstitial spots;
- infiltrating an amount of water or aqueous solution into said solid mold through said empty interstitial spots so that to dissolve said model (1) and to make a mold cavity (8) negative reproducing said model (1);
- infiltrating into the mold cavity (8), through the outer walls of said mold, a second liquid polymer (5); said material (3) having a melting temperature greater than the melting temperature of said second liquid polymer (5) and/or a difference in the Hildebrand solubility parameter greater than or equal to 2 cal^{1/2}cm^{-3/2} with respect to that of said second liquid polymer (5); said step of infiltrating into the mold cavity (8) occurs by depositing the liquid polymer (5) on the outer walls of said mold (7), wherein the second liquid polymer (5) solidifies or hardens in the mold cavity (8) and the device (10) is formed;
- removing said mold by releasing the product obtained and created in said second polymer (5).

2. Printing process according to claim 1, **characterized in that** said model (1) is printed with a soluble polymer (2) selected between thermoplastics or thermosettings.

3. Printing process according to claim 1, **characterized in that** said first soluble polymer (2) comprises one among polyvinyl alcohol, dimethylacrylamide (DMA), methacrylic acid (MA) and its esters, methacrylic anhydride (MAA), polyvinylpyrrolidone (PVP), succinic acid (SAA) and its esters or a combination thereof.

4. Printing process according to claim 1, **characterized in that** said model (1) is completely covered with a layer of said material (3) of a maximum of 2 cm in thickness.

5. Printing process according to claim 1, **characterized in that** said material (3) has a melting temperature greater than the melting temperature of said second liquid polymer (5).

6. Printing process according to any one of claims 1 to 5, **characterized in that** said material (3) is selected among wax, silicone-based elastomers or perfluoropolyethers.

7. Printing process according to any one of preceding claims 1 to 6, **characterized in that** said second liquid polymer (5) is a biocompatible shape-memory polymer.

8. Printing process according to claim 7, **characterized in that** said second biocompatible shape-memory polymer is selected between a one-way biocompatible shape-memory polymer or a two-way biocompatible shape-memory polymer.

9. Printing process according to claim 1, **characterized in that**, in said step of infiltrating into the mold cavity (8) a second liquid polymer (5) through the outer walls of said mold (7), the infiltration occurs by capillarity.

10. Printing process according to any one of preceding claims 1 to 9, **characterized in that** in said step of infiltrating into the mold cavity (8) a second liquid polymer (5) through the outer walls of said mold (7), the mold (7) and said second liquid polymer (5) deposited thereon are subjected to the action of a vacuum source which creates a vacuum inside the mold cavity (8).

11. Printing process according to claim 1, **characterized in that** in said step of infiltrating into the mold cavity (8) a second liquid polymer (5) through the outer walls of said mold (7), the infiltration occurs by injecting said second liquid polymer (5) into said mold cavity (8).

12. Printing process according to any one of preceding claims 1 to 11, **characterized in that**, in said step of removing said mold (7), said mold (7) is immersed into an organic solvent so that to be dissolved.

13. Printing process according to any one of preceding claims 1 to 11, **characterized in that**, in said step of removing said mold (7), said mold (7) is removed mechanically.

14. Printing process according to any one of preceding claims 1 to 11, **characterized in that**, in said step of removing said mold (7), said mold (7) is subjected to the heat generated by a heat source.

## Patentansprüche

1. Verfahren zum hochauflösenden Drucken von vorzugsweise medizinischen Vorrichtungen (10) mit komplexen Geometrien, umfassend die Schritte:
- Drucken eines Modells (1) mit einem dreidimensionalen Druckverfahren unter Verwendung eines dreidimensionalen Druckers; wobei das Modell (1) die herzustellende medizinische Vorrichtung (10) positiv wiedergibt;
- wobei das Modell (1) aus einem ersten wasserlöslichen Polymer (2) oder wässrigen Lösungen gedruckt wird;
- Überziehen des Modells (1) mit einer Schicht aus einem Material (3), das gegenüber einer Lösung unlöslich ist, die in der Lage ist, das erste lösliche Polymer (2) aufzulösen; wobei der Schritt des Überziehens eine massive Formschale (7) erzeugt, die eine leere Zwischenraumstellen umfassende Oberfläche aufweist;
- Infiltrieren einer Menge von Wasser oder wässriger Lösung in die massive Form durch die leeren Zwischenraumstellen, um das Modell (1) aufzulösen und einen Formhohlraum (8) herzustellen, der das Modell (1) negativ wiedergibt;
- Infiltrieren eines zweiten flüssigen Polymers (5) in den Formhohlraum (8) durch die Außenwände der Form, wobei das Material (3) eine Schmelztemperatur aufweist, die höher ist als die Schmelztemperatur des zweiten flüssigen Polymers (5) und/oder einen Unterschied im Hildebrand-Löslichkeitsparameter von mehr als oder gleich 2 cal^{1/2} cm^{-3/2} gegenüber demjenigen des zweiten flüssigen Polymers (5); der Schritt des Infiltrierens in den Formhohlraum (8) durch Auftragen des flüssigen Polymers (5) auf die Außenwände der Form (7) erfolgt, wobei das zweite flüssige Polymer (5) in dem Formhohlraum (8) erstarrt oder aushärtet und dadurch die Vorrichtung (10) gebildet wird;
- Entfernen der Form durch Freigabe des erhaltenen und aus dem zweiten Polymer (5) hergestellten Produkts.

2. Druckverfahren nach Anspruch 1, **dadurch gekennzeichnet, dass** das Modell (1) mit einem löslichen Polymer (2) ausgewählt unter Thermoplasten oder Duroplasten bedruckt wird.

3. Druckverfahren nach Anspruch 1, **dadurch gekennzeichnet, dass** das erste lösliche Polymer (2) eines der folgenden Stoffe umfasst: Polyvinylalkohol, Dimethylacrylamid (DMA), Methacrylsäure (MA) und ihre Ester, Methacrylsäureanhydrid (MAA), Polyvinylpyrrolidon (PVP), Bernsteinsäure (SAA) und ihre Ester oder eine Kombination davon.

4. Druckverfahren nach Anspruch 1, **dadurch gekennzeichnet, dass** das Modell (1) vollständig mit einer Schicht des besagten Materials (3) mit einer Dicke von maximal 2 cm überzogen wird.

5. Druckverfahren nach Anspruch 1, **dadurch gekennzeichnet, dass** das Material (3) eine Schmelztemperatur hat, die höher ist als die Schmelztemperatur des zweiten flüssigen Polymers (5).

6. Druckverfahren nach einem der Ansprüche 1 bis 5, **dadurch gekennzeichnet, dass** das Material (3) unter Wachs, Elastomeren auf Silikonbasis oder Perfluorpolyether ausgewählt ist.

7. Druckverfahren nach einem der vorhergehenden Ansprüche 1 bis 6, **dadurch gekennzeichnet, dass** das zweite flüssige Polymer (5) ein biokompatibles Formgedächtnispolymer ist.

8. Druckverfahren nach Anspruch 7, **dadurch gekennzeichnet, dass** das zweite biokompatible Formgedächtnispolymer unter einem einwegigen biokompatiblen Formgedächtnispolymer oder einem zweiwegigen biokompatiblen Formgedächtnispolymer ausgewählt ist.

9. Druckverfahren nach Anspruch 1, **dadurch gekennzeichnet, dass** in dem Schritt des Infiltrierens eines zweiten flüssigen Polymers (5) in den Formhohlraum (8) durch die Außenwände der Form (7) die Infiltration durch Kapillarität erfolgt.

10. Druckverfahren nach einem der vorhergehenden Ansprüche 1 bis 9, **dadurch gekennzeichnet, dass** in dem Schritt des Infiltrierens eines zweiten flüssigen Polymers (5) in den Formhohlraum (8) durch die Außenwände der Form (7) die Form (7) und das darauf aufgetragene zweite flüssige Polymer (5) der Wirkung einer Vakuumquelle unterworfen werden, die ein Vakuum innerhalb des Formhohlraums (8) erzeugt.

11. Druckverfahren nach Anspruch 1, **dadurch gekennzeichnet, dass** in dem Schritt des Infiltrierens eines zweiten flüssigen Polymers (5) in den Formhohlraum (8) durch die Außenwände der Form (7) die Infiltration durch Einspritzen des zweiten flüssigen Polymers (5) in den Formhohlraum (8) erfolgt.

12. Druckverfahren nach einem der vorhergehenden Ansprüche 1 bis 11, **dadurch gekennzeichnet, dass** in dem Schritt des Entfernens der Form (7) die Form (7) in ein organisches Lösungsmittel eingetaucht wird, um aufgelöst zu werden.

13. Druckverfahren nach einem der vorhergehenden Ansprüche 1 bis 11, **dadurch gekennzeichnet, dass** in dem Schritt des Entfernens der Form (7) die Form (7) mechanisch entfernt wird.

14. Druckverfahren nach einem der vorhergehenden Ansprüche 1 bis 11, **dadurch gekennzeichnet, dass** in dem Schritt des Entfernens der Form (7) die Form (7) einer durch eine Wärmequelle erzeugten Wärme ausgesetzt wird.

## Revendications

1. Méthode d'impression à haute résolution de dispositifs (10), de préférence médicaux, avec des géométries complexes, comprenant les étapes suivantes:
- imprimer un modèle (1) avec une méthode d'impression tridimensionnelle en utilisant une imprimante tridimensionnelle; ledit modèle (1) reproduisant en positif le dispositif médical (10) à réaliser;
- ledit modèle (1) étant imprimé à partir d'un premier polymère hydrosoluble (2) ou de solutions aqueuses;
- recouvrir ledit modèle (1) d'une couche de matériau (3) insoluble à une solution adaptée à dissoudre ledit premier polymère soluble (2); ladite étape de recouvrement réalisant une coque de moule solide (7) pourvue d'une surface comprenant des points interstitiels vides;
- infiltrer une quantité d'eau ou de solution aqueuse dans ledit moule solide à travers lesdits points interstitiels vides de sorte à dissoudre ledit modèle (1) et à réaliser une cavité de moule (8) reproduisant négativement ledit modèle (1);
- infiltrer dans la cavité de moule (8), à travers les parois externes dudit moule, un second polymère liquide (5); ledit matériau (3) ayant une température de fusion supérieure à la température de fusion dudit second polymère liquide (5) et/ou une différence du paramètre de solubilité de Hildebrand supérieure ou égale à 2 cal^{1/2} cm^{-3/2} par rapport à celui dudit second polymère liquide (5); ladite étape d'infiltration dans la cavité de moule (8) se produit en déposant le polymère liquide (5) sur les parois externes dudit moule (7), dans laquelle le second polymère liquide (5) se solidifie ou durci dans la cavité de moule (8) et le dispositif (10) se forme;
- retirer ledit moule en relâchant le produit obtenu et réalisé dans ledit second polymère (5).

2. Méthode d'impression selon la revendication 1, **caractérisée en ce que** ledit moule (1) est imprimé avec un polymère soluble (2) sélectionné parmi les thermoplastiques ou les thermodurcissables.

3. Méthode d'impression selon la revendication 1, **caractérisée en ce que** ledit premier polymère soluble (2) comprend un parmi l'alcool polyvinylique, le diméthylacrylamide (DMA), l'acide méthacrylique (MA) et ses esters, l'anhydride méthacrylique (MAA), la polyvinylpyrrolidone (PVP), l'acide succinique (SAA) et ses esters ou une combinaison des mêmes.

4. Méthode d'impression selon la revendication 1, **caractérisée en ce que** ledit modèle (1) est complètement recouvert d'une couche dudit matériau (3) d'une épaisseur maximale de 2 cm.

5. Méthode d'impression selon la revendication 1, **caractérisée en ce que** ledit matériau (3) a une température de fusion supérieure à la température de fusion dudit second polymère liquide (5).

6. Méthode d'impression selon l'une quelconque des revendications 1 à 5, **caractérisée en ce que** ledit matériau (3) est sélectionné parmi la cire, les élastomères à base de silicone ou les perfluoropolyéthers.

7. Méthode d'impression selon l'une quelconque des revendications précédentes 1 à 6, **caractérisée en ce que** ledit second polymère liquide (5) est un polymère à mémoire de forme biocompatible.

8. Méthode d'impression selon la revendication 7, **caractérisée en ce que** ledit second polymère à mémoire de forme biocompatible est sélectionné parmi un polymère à mémoire de forme biocompatible unidirectionnel ou un polymère à mémoire de forme biocompatible bidirectionnel.

9. Méthode d'impression selon la revendication 1, **caractérisée en ce que**, dans ladite étape d'infiltration d'un second polymère liquide (5) dans la cavité de moule (8) à travers les parois externes dudit moule (7), l'infiltration se produit par capillarité.

10. Méthode d'impression selon l'une quelconque des revendications précédentes 1 à 9, **caractérisée en ce que**, dans ladite étape d'infiltration d'un second polymère liquide (5) dans la cavité de moule (8) à travers les parois externes dudit moule (7), le moule (7) et ledit second polymère liquide (5) déposé sur le même sont soumis à l'action d'une source de vide qui crée un vide à l'intérieur de la cavité de moule (8).

11. Méthode d'impression selon la revendication 1, **caractérisée en ce que**, dans ladite étape d'infiltration d'un second polymère liquide (5) dans la cavité de moule (8) à travers les parois externes dudit moule (7), l'infiltration se produit en injectant ledit second polymère liquide (5) dans ladite cavité de moule (8).

12. Méthode d'impression selon l'une quelconque des revendications précédentes 1 à 11, **caractérisée en ce que**, dans ladite étape de retrait dudit moule (7), ledit moule (7) est immergé dans un solvant organique de sorte à être dissous.

13. Méthode d'impression selon l'une quelconque des revendications précédentes 1 à 11, **caractérisée en ce que**, dans ladite étape de retrait dudit moule (7), ledit moule (7) est retiré mécaniquement.

14. Méthode d'impression selon l'une quelconque des revendications précédentes 1 à 11, **caractérisée en ce que**, dans ladite étape de retrait dudit moule (7), ledit moule (7) est soumis à la chaleur générée par une source de chaleur.
